(19)

**Europäisches Patentamt**
**European Patent Office**
**Office européen des brevets**

(11) **EP 4 702 923 A1**

(12) # EUROPEAN PATENT APPLICATION

(43) Date of publication:
 **04.03.2026 Bulletin 2026/10**

(21) Application number: **25197013.3**

(22) Date of filing: **20.08.2025**

(51) International Patent Classification (IPC):
 ***A61B 5/16*** *(2006.01)* ***G06Q 30/00*** *(2023.01)*
 ***G16H 10/20*** *(2018.01)* ***A61B 5/00*** *(2006.01)*

(52) Cooperative Patent Classification (CPC):
 **A61B 5/165; A61B 5/7405; A61B 5/744;**
 **G06Q 30/0201; G06Q 30/02011; G06Q 30/0251;**
 **G06Q 30/0271; G16H 10/20;** A61B 2503/12

(84) Designated Contracting States:
 **AL AT BE BG CH CY CZ DE DK EE ES FI FR GB**
 **GR HR HU IE IS IT LI LT LU LV MC ME MK MT NL**
 **NO PL PT RO RS SE SI SK SM TR**
 Designated Extension States:
 **BA**
 Designated Validation States:
 **GE KH LA MA MD TN**

(30) Priority: **29.08.2024 IN 202421065329**

(71) Applicant: **Tata Consultancy Services Limited**
 **Maharashtra (IN)**

(72) Inventors:
 • **PANDHARIPANDE, MEGHNA ABHISHEK**
  **400021 Mumbai (IN)**
 • **KOPPARAPU, SUNIL KUMAR**
  **400601 Thane (IN)**
 • **DOKE, PANKAJ HARISH**
  **400021 Mumbai (IN)**

(74) Representative: **Goddar, Heinz J.**
 **Boehmert & Boehmert**
 **Anwaltspartnerschaft mbB**
 **Pettenkoferstrasse 22**
 **80336 München (DE)**

(54) **METHOD AND SYSTEM FOR ASSESSING AN ALTERED EMOTIONAL STATE**

(57) This disclosure relates generally to a method and system for assessing an altered emotional state under the influence of a sensory stimulus. The sensory stimulus are known to have a sub-conscious influence on the emotional state of a person. This is effectively utilized in businesses to improve customer experience, and thus increasing sales. State-of-the-art methods assesses the altered brain state by measuring one or more physiological parameters using fairly complex apparatuses like electroencephalogram (EEG). The disclosed method involves assessing (i) a pre-emotional state before exposing a subject to the sensor stimulus, and (ii) a post-emotional state after exposing the subject to the sensory stimulus. The altered emotional state of the subject is obtained by a vector difference between the pre-emotional state and the post-emotional state which is an angular difference calculated as a cosine of an angle between the pre-emotional state and post-emotional state.

FIG. 2

EP 4 702 923 A1

**Description**

CROSS-REFERENCE TO RELATED APPLICATIONS AND PRIORITY

The present application claims priority to Indian application no. 202421065329, filed on August 29, 2024.

TECHNICAL FIELD

The disclosure herein generally relates to an intelligent automation in influencer marketing, and, more particularly, a method and system for assessing emotional state of a subject by utilizing a sensory stimulus to influence customer experience.

BACKGROUND

Emotions have an influence on consumer decision-making. The emotional state perceived by a consumer at a particular instance has a correlation with a set of cognitive appraisals that become the driver to steer the emotions for decision-making through a nuanced psychological mechanisms. Moreover, the emotional state can be considered to have a long and a short-term emotional state just like the long and short-term memory of cognitive models. The emotions influence consumer to decide whether to purchase a product or not. The surroundings associated with the product also creates a sub-conscious influence on the emotional state and thus empowered to alter/ influence consumer decision-making. The sub-conscious influence on the emotional state is ensued through an external stimuli perceived by human senses. The emotions can be manipulated through an external stimuli. Therefore, marketing often exploit the potential of altering emotional state of the consumer by providing an external stimulus through the human senses.

The distinctive and characteristic odors associated with a product or a place acts as an olfactory stimulus and impact one's cognitive and affective responses, attitudes and perceptions, as well as memory and behaviors. Similarly, a same person can appreciate one type of music in some state of mind, but the same music may not be appreciated by the same person in another state of mind. Nevertheless, an aural stimulus has been realized as a game changer and contributes positively in altering the emotional state of the person. Likewise, colors influence perceptions. Colors have qualities that can cause certain emotions in people. Colors have been widely used in marketing and branding as a visual stimulus. Marketers see color as an important factor, since color can influence a consumers' emotions and perceptions about goods and services. Therefore, various sensory stimulus have the potential to alter an emotional state of the person and hence widely utilized to influence the consumer.

Detecting the emotional state using biological brain signals utilizes one or more sophisticated techniques such as Electroencephalography (EEG), Magnetic Resonance Imaging (MRI), functional MRI (fMRI), cranial ultrasound, to name a few and have been used to examine the brain functions. Of these, EEG is a non-invasive technique and provides information about temporal measure of responses, of the central nervous system. The EEG power spectrum is generally studied as a set of well-known frequency bands, namely, delta ($\delta$, 0 - 4 Hz), theta ($\theta$, 4 - 8 Hz), alpha ($\alpha$, 8 - 13 Hz), beta ($\beta$, 13-30 Hz), and gamma ($\gamma$, 30-50 Hz). Additionally, each of these bands can be correlated with different brain states. Brain activity signals are captured by EEG using a set of electrodes placed along the scalp using the international 10-20 system. However, despite these advantages, potential applications remain limited due to the high cost, low mobility, and long preparation times associated with high-density (large number of electrodes) EEG recording systems. The EEGs are extremely reliable in terms of identifying one or more disorder associated with brain-functioning. Low-cost consumer EEG headsets are also available on the market which are not as complex as the conventional EEG recording systems. The usage of these head-sets demonstrate relative ease of identifying brain states, although low levels of accuracy, sensitivity, and specificity and the necessity of training are likely issues have been reported so far. However, the EEG alone may not be sufficient to assess dynamic emotions associated with a person that changes frequently with the change in one or more sensory stimuli and would need to supplement with one or more tools for the emotional state assessment.

SUMMARY

Embodiments of the present disclosure present technological improvements as solutions to one or more of the above-mentioned technical problems recognized by the inventors in conventional systems. For example, in one embodiment, method of assessing an altered emotional state is provided. The method includes receiving a first self-reported emotional state of a subject by exposing the subject to a semiotic instrument. The semiotic instrument comprises a plurality non-verbal cues expressing a plurality of emotions, and wherein each non-verbal cue of the plurality of non-verbal cues is coupled with a strength indicator. The non-verbal cue as well as the strength indicator together illustrates one or more emotions along with an intensity of the emotions in terms of the strength indicator. The method further includes processing the first self-reported emotional state of the subject using a pre-defined metric to generate a first semiotic score of the subject. The pre-defined metric involves a weights assigned to each non-verbal cue to differentiate a plurality of strength indicator associated with the non-verbal cues. The method further includes obtaining a first cognitive score of the subject by presenting to the subject at least one cognitive instrument. A response of the subject while interacting with the at least one cognitive instrument is processed to generate the first cognitive score of the subject. The at least one cognitive instrument is (i) a linguistic instrument, (ii) an aural instrument, and (iii) a visual instrument. The linguistic instrument comprises a set of words largely used to explain one or more emotional state and is derived by using a LLM to address language biases and wider acceptability. The aural instrument comprises a set of chords representing one or more music that can associate with a plurality of emotions of the subject. The visual instrument comprises a set of color palette that can associate with the plurality of emotions of the subject. The method further includes assessing a pre-emotional state of the subject by aggregating the first semiotic score and the first cognitive score. The first cognitive score is a score of at least one of a linguistic score obtained from the linguistic instrument, an aural score obtained from the aural instrument, or a visual score obtained from the visual instrument. The first cognitive score is a combination of at least two of the cognitive instruments. The first cognitive score is a combination of all three cognitive instruments. The method further includes providing at least one sensory input. The sensory input is generated by exposing the subject to stimulate at least one of a plurality of human senses. The sensory input is perceived by the subject to experience a state of emotional variability. The stimulation of the plurality of human senses includes exposing the subject to an odor, or by providing a tactile stimulus, or by administering an edible substance having a distinct taste. The method further includes receiving a second self-reported emotional state of the subject by exposing the subject to the semiotic instrument. The second self-reported emotional state represents a state of the subject post-sensory stimulus. The same semiotic instrument that is administered in identifying the pre-emotional state that comprises the plurality of non-verbal cues expressing the plurality of emotions is used. The method further includes processing the second self-reported emotional state of the subject using the pre-defined metric to generate a second semiotic score of the subject. The method further includes obtaining a second cognitive score of the subject by presenting to the subject at least one cognitive instrument. The response of the subject while interacting with the at least one cognitive instrument post sensory stimulus is processed to generate the second cognitive score of the subject. The at least one cognitive instrument is (i) the linguistic instrument, (ii) the aural instrument, and (iii) the visual instrument. The method further includes assessing a post-emotional state of the subject by aggregating the second semiotic score and the second cognitive score. The method further includes estimating an altered emotional state of the subject by obtaining a vector difference between the pre-emotional state and the post-emotional state. The vector difference between the pre-emotional state and the post-emotional state is an angular difference calculated as a cosine of an angle between the pre-emotional state and post-emotional state, and represents the altered emotiona state of the subject under the influence the sensory stimulus.

In another aspect, a system for assessing an altered emotional state is provided. The system includes at least one memory storing programmed instructions; one or more Input /Output (I/O) interfaces; and one or more hardware processors, an emotional state assessment module, a pre-emotional state sub-module, a post-emotional state sub-module, a sensory stimuli analysis sub-module, and an EEG data processing sub-module operatively coupled to a corresponding at least one memory, wherein the system is configured to receive a first self-reported emotional state of a subject by exposing the subject to a semiotic instrument. The semiotic instrument comprises a plurality of non-verbal cues expressing a plurality of emotions, and wherein each non-verbal cue of the plurality of non-verbal cues is coupled with a strength indicator. The non-verbal cue as well as the strength indicator together illustrates one or more emotions along with an intensity of the emotions in terms of the strength indicator. Further, the system is configured to process the first self-reported emotional state of the subject using a pre-defined metric to generate a first semiotic score of the subject. The pre-defined metric involve weights assigned to each non-verbal cue to differentiate a plurality of strength indicator associated with the non-verbal cues. Further, the system is configured to obtain a first cognitive score of the subject by presenting to the subject at least one cognitive instrument. A response of the subject while interacting with the at least one cognitive instrument is processed to generate the first cognitive score of the subject. The at least one cognitive instrument is (i) a linguistic instrument, (ii) an aural instrument, and (iii) a visual instrument. The linguistic instrument comprises a set of words largely used to explain one or more emotional state and is derived by using a LLM to address language biases and wider acceptability. The aural instrument comprises a set of chords representing one or more music that can associate with a plurality of emotions of the subject. The visual instrument comprises a set of color palette that can associate with the plurality of emotions of the subject. Further, the system is configured to assess a pre-emotional state of the subject by aggregating the first semiotic score and the first cognitive score. The First cognitive score is a score of at least one of a linguistic score obtained from the linguistic instrument, an aural score obtained from the aural instrument, or a visual score obtained from the visual instrument. The first cognitive score is a combination of at least two of the cognitive instruments. The first cognitive score is a combination of all three cognitive instruments. Further, the system is configured to provide at least one sensory input. The sensory input is generated by exposing the subject to stimulate at least one of a plurality of human senses. The sensory input is perceived by the subject to experience a state of emotional variability. The stimulation of the plurality of human senses includes exposing the subject to an odor, or by providing a tactile stimulus, or by administering an edible substance having a distinct taste. Further, the system is configured to receive a second self-reported emotional state of the subject by exposing the subject to the semiotic instrument. The second self-reported emotional state represents a state of the subject post-sensory stimulus. The same semiotic instrument that is administered in identifying the pre-emotional state that comprises the plurality non-verbal cues expressing the plurality of emotions is used. Further, the system is configured to process the second self-reported emotional state of the subject using the pre-defined metric to generate a second semiotic score of the subject. Further, the system is configured to obtain a second cognitive score of the subject by presenting to the subject at least one cognitive instrument. The response of the subject while interacting with the at least one cognitive instrument post sensory stimulus is processed to generate the second cognitive score of the subject. The at least one cognitive instrument is (i) the linguistic instrument, (ii) the aural instrument, and (iii) the visual instrument. Further, the system is configured to assess a post-emotional state of the subject by aggregating the second semiotic score and the second cognitive score. Further, the system is configured to estimate an altered emotional state of the subject by obtaining a vector difference between the pre-emotional state and the post-emotional state. The vector difference between the pre-emotional state and the post-emotional state is an angular difference calculated as a cosine of an angle between the pre-emotional state and post-emotional state, and represents the altered emotiona state of the subject under the influence the sensory stimulus.

**In** yet another aspect, a computer program product including a non-transitory computer-readable medium embodied therein a computer program for assessing an altered emotional state is provided. The computer readable program, when executed on a computing device, causes the computing device to receive a first self-reported emotional state of a subject by exposing the subject to a semiotic instrument. The semiotic instrument comprises a plurality of non-verbal cues expressing a plurality of emotions, and wherein each non-verbal cue of the plurality of non-verbal cues is coupled with a strength indicator. The non-verbal cue as well as the strength indicator together illustrates one or more emotions along with an intensity of the emotions in terms of the strength indicator. The computer readable program, when executed on a computing device, causes the computing device to process the first self-reported emotional state of the subject using a pre-defined metric to generate a first semiotic score of the subject. The pre-defined metric involve weights assigned to each non-verbal cue to differentiate a plurality of strength indicator associated with the non-verbal cues. The computer readable program, when executed on a computing device, causes the computing device to obtain a first cognitive score of the subject by presenting to the subject at least one cognitive instrument. A response of the subject while interacting with the at least one cognitive instrument is processed to generate the first cognitive score of the subject. The at least one cognitive instrument is (i) a linguistic instrument, (ii) an aural instrument, and (iii) a visual instrument. The linguistic instrument comprises a set of words largely used to explain one or more emotional state and is derived by using a LLM to address language biases and wider acceptability. The aural instrument comprises a set of chords representing one or more music that can associate with a plurality of emotions of the subject. The visual instrument comprises a set of color palette that can associate with the plurality of emotions of the subject. The computer readable program, when executed on a computing device, causes the computing device to assess a pre-emotional state of the subject by aggregating the first semiotic score and the first cognitive score. The First cognitive score is a score of at least one of a linguistic score obtained from the linguistic instrument, an aural score obtained from the aural instrument, or a visual score obtained from the visual instrument. The first cognitive score is a combination of at least two of the cognitive instruments. The first cognitive score is a combination of all three cognitive instruments. The computer readable program, when executed on a computing device, causes the computing device to provide at least one sensory input. The sensory input is generated by exposing the subject to stimulate at least one of a plurality of human senses. The sensory input is perceived by the subject to experience a state of emotional variability. The stimulation of the plurality of human senses includes exposing the subject to an odor, to a music, or to colors, or by providing a tactile stimulus, or by administering an edible substance having a distinct taste. The computer readable program, when executed on a computing device, causes the computing device to receive a second self-reported emotional state of the subject by exposing the subject to the semiotic instrument. The second self-reported emotional state represents a state of the subject post-sensory stimulus. The same semiotic instrument that is administered in identifying the pre-emotional state that comprises the plurality non-verbal cues expressing the plurality of emotions is used. The computer readable program, when executed on a computing device, causes the computing device to process the second self-reported emotional state of the subject using the pre-defined metric to generate a second semiotic score of the subject. The computer readable program, when executed on a computing device, causes the computing device to obtain a second cognitive score of the subject by presenting to the subject at least one cognitive instrument. The response of the subject while interacting with the at least one cognitive instrument post sensory stimulus is processed to generate the second cognitive score of the subject. The at least one cognitive instrument is (i) the linguistic instrument, (ii) the aural instrument, and (iii) the visual instrument. The computer readable program, when executed on a computing device, causes the computing device to assess a post-emotional state of the subject by aggregating the second semiotic score and the second cognitive score. The computer readable program, when executed on a computing device, causes the computing device to estimate an altered emotional state of the subject by obtaining a vector difference between the pre-emotional state and the post-emotional state. The vector difference between the pre-emotional state and the post-emotional state is an angular difference calculated as a cosine of an angle between the pre-emotional state and post-emotional state, and represents the altered emotiona state of the subject under the influence the sensory stimulus.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system 100 for assessing and altered emotional state influenced by a plurality of sensory stimulus, according to some embodiments of the present disclosure.
FIG. 2 illustrates architectural overview of system 100 for the assessment of the altered emotional state under the influence of the plurality of sensory stimulus, according to some embodiments of the present disclosure.
FIGS. 3A-3B are flow diagrams of an illustrative method 400 for assessing altered emotional state using the system of FIG. 1, according to some embodiments of the present disclosure.
FIG. 4 illustrates a semiotic instrument utilized to assess a pre-emotional state and a post emotional state, according to some embodiments of the present disclosure.
FIG. 5 illustrates a linguistic instrument utilized to assess the pre-emotional state and the post emotional state, according to some embodiments of the present disclosure.
FIG. 6 illustrates an aural instrument utilized to assess the pre-emotional state and the post emotional state, according to some embodiments of the present disclosure.
FIG. 7 illustrates a visual instrument utilized to assess a pre-emotional state and a post emotional state, according to some embodiments of the present disclosure.

DETAILED DESCRIPTION

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Embodiments herein provide a method for assessing an altered emotional state under the influence of a sensory stimulus. The sensory stimulus are known to have a sub-conscious influence on the emotional state of a person. This is effectively utilized in businesses to improve customer experience, and thus increasing sales. State-of-the-art methods assesses the altered brain state by measuring one or more physiological parameters using fairly complex apparatuses like electroencephalogram (EEG). The disclosed method involves assessing (i) a pre-emotional state before exposing a subject to the sensor stimulus, and (ii) a post-emotional state after exposing the subject to the sensory stimulus. The altered emotional state of the subject is obtained by a vector difference between the pre-emotional state and the post-emotional state which is an angular difference calculated as a cosine of an angle between the pre-emotional state and post-emotional state.

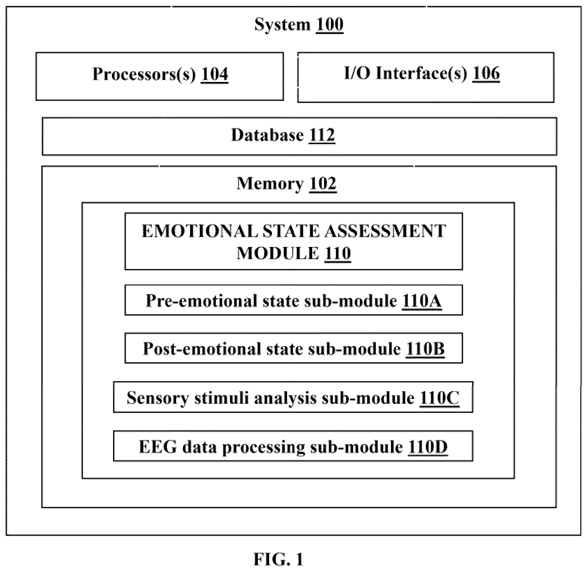

Referring now to the drawings, and more particularly to FIG. 1 through FIG. 7, where similar reference characters denote corresponding features consistently throughout the figures, there are shown preferred embodiments, and these embodiments are described in the context of the following exemplary system and/or method.

FIG. 1 illustrates an exemplary block diagram of a system 100 for assessing and altered emotional state influenced by a plurality of sensory stimulus, according to some embodiments of the present disclosure.

In an embodiment, the system 100 includes a processor(s) 104, communication interface device(s) 106, alternatively referred as input/output (I/O) interface(s) 106, and one or more data storage devices or a memory 102 operatively coupled to the processor(s) 104. The system 100 with one or more hardware processors is configured to execute functions of one or more functional blocks of the system 100. Referring to the components of system 100, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, and the like. The I/O interface(s) 106 can include a variety of software and hardware interfaces, for example, a web interface, a graphical user interface to display the generated target images and the like and can facilitate multiple communications within a wide variety of networks N/W and protocol types, including wired networks, for example, LAN, cable, etc., and wireless networks, such as WLAN, cellular and the like. In an embodiment, the I/O interface (s) 106 can include one or more ports for connecting to number of external devices or to another server or devices. The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 102 includes an emotional state assessment module 110.

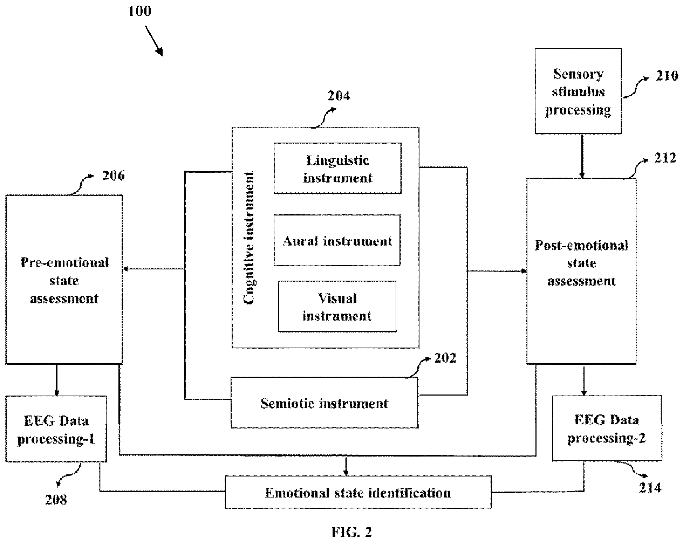

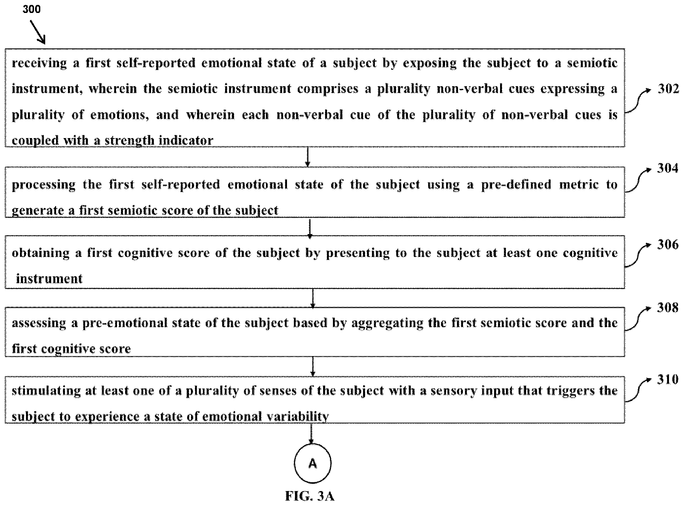

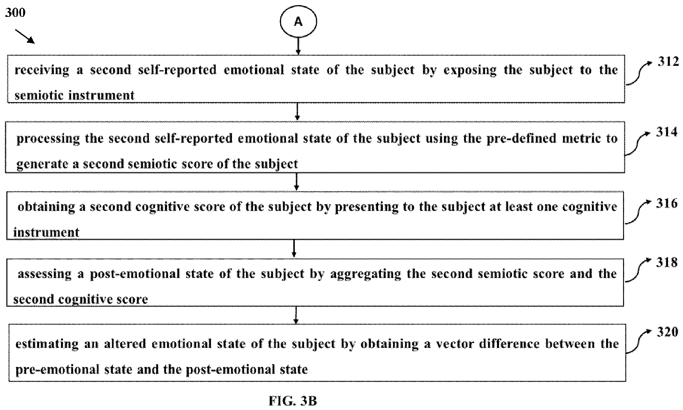

The emotional state assessment module 110 comprises a pre-emotional state sub-module 110A, and a post-emotional state sub-module 110B that separately processes the emotional states before and after sensory stimulus respectively. The emotional state assessment module 110 further comprises a sensory stimuli analysis sub-module 110C, and an EEG data processing sub-module 110D. The pre-emotional state sub-module 110A utilizes a semiotic instrument and a plurality of cognitive instruments to analyses an emotional state of a subject undergoing assessment of an altered emotional state. The post-emotional state sub-module 110B also utilizes the same semiotic instrument and the plurality of cognitive instruments as used in pre-emotional state analysis to assess the emotional state of the subject post sensory stimuli. The difference in the pre-emotional state and the post-emotional state is utilized in understanding the altered emotional state of the subject. The sensory stimuli analysis sub-module processes the response of the plurality of sensory stimulus given to the subject. The EEG data processing sub-module 110D receives EEG signals of the subject during the pre-emotional state analysis and the post-emotional state analysis. The emotional state assessment module 110 further comprises of a plurality of sub-modules that includes programs or coded instructions that supplement applications or functions performed by the system 100 for assessing altered emotional state of the subject under the influence of the sensory stimulus, being provided by the system 100. The modules, amongst other things, can include routines, programs, objects, components, and data structures, which perform particular tasks or implement particular abstract data types. The modules may also be used as signal processor(s), node machine(s), logic circuitries, and/or any other device or component that manipulates signals based on operational instructions. Further, the modules can be used by hardware, by computer-readable instructions executed by the one or more hardware processors 104, or by a combination thereof. The modules may include computer-readable instructions that supplement applications or functions performed by the system 100. The integrated architecture of the plurality of modules and sub-modules depicting the architectural overview of the system 100 is shown in FIG. 2 and explained in conjunction with a method flow diagram depicted in FIG. 3A-3B. Further, the memory 102 may comprise information pertaining to input(s)/output(s) of each step performed by the processor(s) 104 of the system100 and methods of the present disclosure. Further, the memory 102 includes a database 108. The database (or repository) 108 may include a plurality of abstracted piece of code for refinement and data that is processed, received, or generated as a result of the execution of the plurality of modules. The system 100 comprises database 112 shown as internal to the system 100. It will be noted that, in alternate embodiments, the database 112 can also be implemented external to the system 100. The external database is communicatively coupled to the system 100. The data contained within such an external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS).

FIG. 2 illustrates architectural overview of system 100 for the assessment of the altered emotional state under the influence of the plurality of sensory stimulus, according to some embodiments of the present disclosure.

As illustrated in FIG. 2, the system 100 for assessing an altered emotional state under the influence of the sensory stimulus comprises three major stages (i) assessment of present emotional state of the subject without any influence of the sensory stimulus (herein referred as 'pre-emotional state'), (ii) an act of modifying the present emotional state via one or more sensory stimulus, and (iii) assessment of the post sensory stimulus emotional state (herein referred as 'post-emotional state').

The assessment of pre-emotional state 206 before the sensory stimulus and the assessment of post-emotional state 212 of the subject after the sensory stimulus involves administration of a plurality of instruments. The plurality of instruments includes a semiotic instrument 202, and the cognitive instrument 204. A first self-reported emotional state of a subject is generated by exposing the subject to the semiotic instrument 202. The semiotic instrument 202 comprises a plurality of non-verbal cues expressing a plurality of emotions. Each non-verbal cue of the plurality of non-verbal cues is coupled with a strength indicator. The first self-reported emotional state of the subject is processed by the system 100 to generate a first semiotic score of the subject. Further, at least one cognitive instrument 204 is administered and the response of the subject to the semiotic instrument 202, and the cognitive instrument 204 is processed by the system 100 to identify the pre-emotional state 206. A plurality of cognitive instruments are designed to assess the emotional state of the subject. The plurality of cognitive instruments includes (i) a linguistic instrument, (ii) an aural instrument, and (iii) a visual instrument. Administering the at least one cognitive instrument 204 to the subject, a first cognitive score of the subject is generated. The pre-emotional state 206 of the subject is thus assessed by aggregating the first semiotic score and the first cognitive score. The pre-emotional state 206 of the subject is represented below as

$$\text{Pre-emotional state} = SI_1 + CI_1 \qquad\qquad (1)$$

where, $SI_1$ is a first semiotic score, and $CI_1$ is first cognitive score as an aggregation of the linguistic instrument, the aural instrument and the visual instrument and is represented as $(LI_1 + UI_1 + VI_1)$, and where $LI_1$ is $(a_1e_1+.................a_ne_n)_{pre}$, $UI_1$ is $(b_1e_1 + ......... b_ne_n)_{pre}$, and $VI_1$ is $(c_1e_1 + ......... c_ne_n)_{pre}$, and where $e_1$ to $e_n$ are a number of self-reported emotions from a plurality of emotions presented in the linguistic instrument, the aural instrument and the visual instrument, and $a_1 = 1$, $a_2 = 0.5$, $a_3 = 0.25$, .......and $a_n = n_{1/2}$ are the half-life of each of the plurality of emotions.

Similarly, the assessment of post-emotional state 212 is performed after providing the sensory stimulus to the subject. The assessment of post-emotional state 212 of the subject after the sensory stimulus involves administration of the plurality of same instruments, the semiotic instrument 202, and the cognitive instrument 204. A second self-reported emotional state of a subject is generated by exposing the subject to the semiotic instrument 202 after sensory stimulus. The second self-reported emotional state of the subject is processed by the system 100 to generate a second semiotic score of the subject. Further, the at least one cognitive instrument 204 is administered and the response of the subject to the semiotic instrument 202, and the cognitive instrument 204 is processed by the system 100 to identify the post-emotional state 212. By administering the at least one cognitive instrument 204 to the subject, a second cognitive score of the subject is generated. The post-emotional state 212 of the subject is thus assessed by aggregating the second semiotic score and the second cognitive score. The post-emotional state 212 of the subject is calculated as:

$$\text{Post-emotional state} = SI_2 + CI_2 \qquad\qquad (2)$$

where, $SI_2$ is a second semiotic score, and $CI_2$ is second cognitive score as an aggregation of the linguistic instrument, the aural instrument and the visual instrument and is represented as $(LI_2 + UI_2 + VI_2)$, and where $LI_1$ is $(a_1e_1+.................a_ne_n)_{post}$, $UI_1$ is $(b_1e_1 + ......... b_ne_n)_{post}$, and $VI_1$ is $(c_1e_1 + ......... c_ne_n)_{post}$, and where $e_1$ to $e_n$ are a number of self-reported emotions from a plurality of emotions presented in the linguistic instrument, the aural instrument and the visual instrument, and $a_1 = 1$, $a_2 = 0.5$, $a_3 = 0.25$, .......and $a_n = n_{1/2}$ are the half-life of each of the plurality of emotions.

To validate the results obtained by the assessment of the pre-emotional state 206 and the post-emotional state 212 through the semiotic instrument 202 and the at least one cognitive instrument 206, electrical activity of subject's brain are recorded using an Electroencephalogram (EEG) headset. A commercially available, low-cost, 4-channel wearable EEG band is utilized to acquire continuous EEG data, sampled at 256 Hz. The EEG headband is placed on the subject's forehead (closer to the eyebrows); the EEG signal is verified for stability using museLSL live display. The EEG is recorded from AF7, AF8, TP9, and TP10 channels for 30 sec to assess the present emotional state of the subject. While the pre-emotional state 206 is assessed by aggregating the first semiotic score and the first cognitive score, the EEG measured the electrical activity in the brain. Therefore, assessment of the pre-emotional state 206 is done by a combined assessment of the semiotic instrument 202 and the cognitive instrument 204, and a validation of the pre-emotional state 206 is done through the EEG data processing 208. Similarly, assessment of the post-emotional state 212 is done by a combined assessment of the semiotic instrument 202 and the cognitive instrument 204, and a validation of the post-emotional state 212 is done through the EEG data processing 214.

FIGS. 3A-3B are flow diagrams of an illustrative method 300 for assessing altered emotional state using the system of FIG. 1, according to some embodiments of the present disclosure.

The steps of method 300 of the present disclosure will now be explained with reference to the components or blocks of the system 100 as depicted in FIG. 1 through FIG. 3B. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

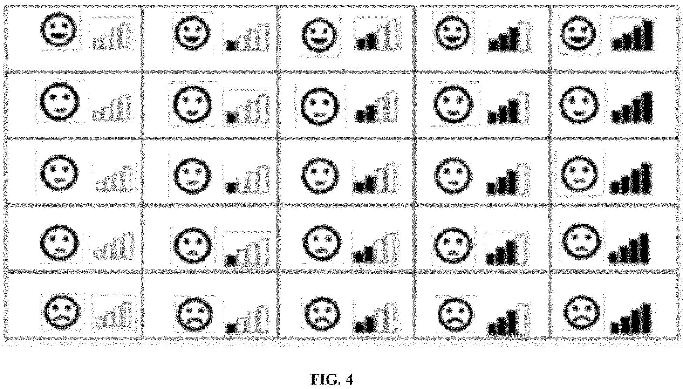

At step 302 of the method 300, the one or more hardware processors 104 are configured to receive a first self-reported emotional state of a subject by exposing the subject to the semiotic instrument 202. The semiotic instrument 202 comprises a plurality of non-verbal cues expressing a plurality of emotions, and wherein each non-verbal cue of the plurality of non-verbal cues is coupled with a strength indicator. The semiotic instrument 202 is designed as a $5 \times 5$ visual probe grid as shown in FIG. 4 that allows the subject to self-report their emotional state. The semiotic instrument 202 comprises a plurality of non-verbal cues expressing a plurality of emotions, and wherein each non-verbal cue of the plurality of non-verbal cues is coupled with a strength indicator. While administering the semiotic instrument 202, the subject is instructed to choose at least one out of 25 visual depictions of a combination of emoji along with the strength indicator. The self-reported emotional state is considered as a pre-emotional state and is expressed as an 'affect'. Emotional valence and arousal are considered as principal dimensions of the affect. The emotional valence is a characteristic that describes the extent to which an emotion is positive or negative. Emotional valence is one of the two dimensions of the emotional state. A state of arousal being the other dimension refers to the strength associated with the emotional state. In the semiotic instrument 202, depiction of various emotional state are presented in the form of a set of emojis bearing resemblance to a plurality of 'smileys'. The rationale of incorporating the set of emojis is to utilize widely known and acceptable tools as well as its prevalence in social media applications. It allows and encourages people to express themselves as emojis. Each emoji is accompanied by a visual imagery of 'signal-strength' to self-report a strength of an emotion. The strength of the emotion is the state of arousal. Similar to the smiley emoji, an icon of the signal strength has a common-ground amongst the subjects. By visually placing the smiley as well as the signal-strength icons in close proximity to each other, a singular entity is obtained that describes the emotional state of the subject. This semiotic instrument as the singular entity attempts to nudge the subject and let the subject communicate his emotional state. The scale of the strength of emotion goes from a 'no-bar-lit' to a 'all-four-bar-lit' conditions. **In** the 'no-bar-lit' condition, the associated emoji is to be interpreted as a lowly expressed actual depiction of the emotional state of the subject wherein the emoji alone is to be inferred as the valence state and is devoid any indication of the state of arousal. **In** the 'all-four-bar-lit' condition, the associated emoji is interpreted as a highly expressed actual depiction of the emotional state of the subject wherein emoji is interpreted as the state of valance and the "all-four-bar-lit" is interpreted as the state of arousal.

At step 304 of the method 300, the one or more hardware processors 104 are configured to process a first self-reported emotional state of the subject using a pre-defined metric to generate a first semiotic score ($M_{pre-emotional}$). Based on response of the subject to the semiotic instrument 202, the first semiotic score is calculated that can best describe the pre-emotional state 206 of the subject expressed in terms of one out of 25 entities (the non-verbal cue + the strength indicator) depicted in the semiotic instrument 202. The metric for the semiotic instrument when odor is administered as stimuli is represented as:

$$\text{First semiotic score or } M_{pre-emotional} = \{Si, Wi\} \qquad (3)$$

where $S_i$= {MH, H, N, S,MS} and S represents one or more emotional states, such as MH is more happy, H is happy, N is neutral, S is sad and MS is more sad],

W = {0,25,50,75,100} where W represents intensity given to each emotional state.
M = {Si,Wi} [Si - one element of the set Si, Wi - one element of the set W]
M {Si, Wi} - score from the metric
Example: M{N,75} means emotions is quantified as neutral with 75% strength.

At step 306 of the method 300, the one or more hardware processors 104 are configured to obtain a first cognitive score of the subject by presenting to the subject at least one cognitive instrument. A response of the subject while interacting with the at least one cognitive instrument is processed to generate the first cognitive score of the subject, and wherein the at least one cognitive instrument is (i) a linguistic instrument, (ii) an aural instrument, and (iii) a visual instrument.

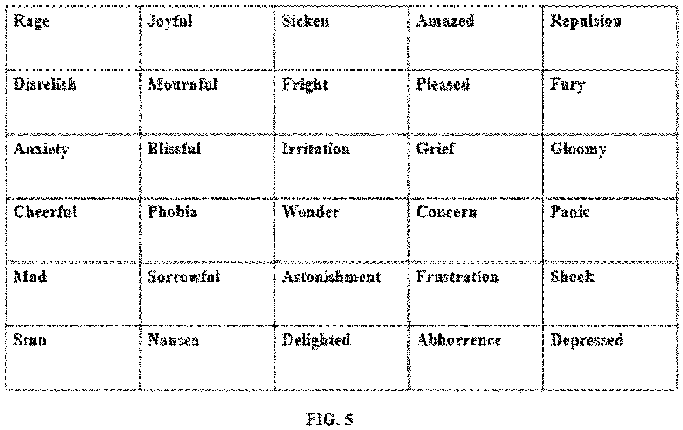

The linguistic instrument is based on word-association with respect to a depiction of various emotional states. The linguistic instrument is designed considering a common-ground in terms of vocabulary and a semantic understanding and overcoming biases. The linguistic instrument is designed using a Generative AI tool ( i.e. a Large Language Model (LLM)). The LLM is prompted to generate a set of words which best represents the six emotions (happiness, surprise, sadness, disgust, fear, and anger). The LLM generated word list is further pruned by removing words which would be considered difficult to interpret by the subjects. Finally, the linguistic instrument having LLM prompted curated as well as manually curated set of words is shown in FIG. 5. Therefore, to design the linguistic instrument, following steps are performed:

(a) Prompt a LLM with a set of words suitable to describe a plurality of emotions;
(b) Retrieve a list of words generated by the LLM in response to the set of words prompted.
(c) Manually curate the list of words retrieved by the LLM, wherein a subjective assessment of the list of words is performed based on wider understanding and acceptability.
(d) Randomize the list of words to arrange in a tabular form, wherein a table has one or more rows and one or more columns. The randomization allows the subject to explore a word-scape and choose one or more words from the table that represents a current emotional state of the subject.

In the linguistic instrument, the set of words, thus, are randomized on a $6 \times 5$ grid. The randomization would allow the subject to explore the word-scape and choose one or more words, as a ranked list, which best represents their pre-emotional state. The subject is instructed to choose at least one word that suits best to describe the self-reported emotional state. In case the subject choose 2-3 words describing his self-reported emotional state, then the subject is prompted to rank the chosen words. To calculate the score of assessing the subject with the linguistic instrument, the set of words by the subject are converted into a 6-D vector, $[e_1, e_2, e_3, e_4, e_5, e_6$, where $e_1$ = happy, $e_2$ = surprise, $e_3$ = sad, $e_4$ = disgust, es = fear, and $e_6$ = anger. When a subject select k(<< 30) number of words, namely, $W = \{z_1, z_2, \cdots, z_k\}$ from the list of 30 words mentioned in the FIG.5, then the chosen list of words W is converted to an emotion vector using an Algorithm 1 presented below. Therefore, $E = w_2ev(W)$, where E is the vector representation of the words (W) chosen by the subject. The vector E is chosen to represent a self-assessed emotional state of the subject.

## Algorithm 1 $E = w2ev(W)$: words to emotion vector

1: Let $W = \{z_1, z_2, \cdots, z_k\}$ be the $k$ words chosen by the subject as a ranked list

2: Let $E = \mathbf{0} \cdot [\bar{e}_1, \bar{e}_2, ..., \bar{e}_6]$ be the *null* emotion vector

3: Let $p \leftarrow 1$

4: for $z \in \{z_1, z_2, \cdots, z_k\}$ do

5: if $z \in \bar{e}_i$ then 6: ## word $z$ represents emotion $e_i$

7: $\quad u = \left(\frac{1}{2}\right)^{(p-1)}$

8: ## Weight is $1, \frac{1}{2}, \frac{1}{4}$ for the $1^{st}$, $2^{nd}$ and $3^{rd}$ words

9: $\quad E \leftarrow E + u\bar{e}_i$

10: end if

11: $p \leftarrow p + 1$

12: end for 13: return $E$

A linguistic score of the pre-emotional state ($LI_1$) is calculated by assessing the subject with the linguistic element as:

$$LI_1 = (a_1e_1 + \ldots\ldots\ldots\ldots a_ne_n)_{pre} \qquad (4)$$

where $e_1$ to $e_n$ are a number of self-reported emotions from the plurality of emotions presented in the linguistic instrument, and $a_1$ = 1, $a_2$ = 0.5, $a_3$ = 0.25, .......and $a_n$ = $n_{1/2}$ are the half-life of each of the plurality of emotions

Therefore, the first cognitive score is calculated using the linguistic instrument by a three-stage process comprising:

(a) A first stage, where the subject is administered with the linguistic instrument.
(b) A second stage, where a response is captured from the subject wherein the response comprises (i) one or more words selected from the linguistic instrument that describes the current emotional state of the subject, and (ii) an assigned rank to each word from the one or more words selected from the linguistic instrument to obtain one or more ranked words.
(c) A third-stage, where the one or more ranked words are processed to calculate the cognitive score of the subject.

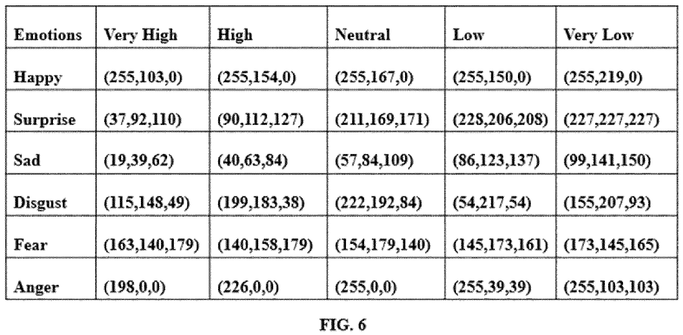

The visual instrument is designed on the basis of color theory that associates a specific color with a specific emotional state. Gohar. Et. al (2008) has demonstrated that color is an important perceptual feature of six basic emotions: anger, disgust, fear, happiness, sadness, and surprise. The color red is most associated with anger, green with disgust, black with fear, yellow with happiness, blue with sadness, and bright with surprise. Moller et al. (2009) found that the color red is positively associated with negative words, as well as with words specific to failure. Young, Elliot, Feltman, and Ambady (2013) examined the ability of the color red to influence the identification of negative facial expressions, specifically anger and fear. Based on the fact that colors have an association with various emotional states as well as they influence the present emotional state of the subject, the visual instrument is designed considering six basic emotions (happy, surprise, sad, disgust, fear and anger). Also, to precisely assess the intensity of the present emotional state of the subject, each of the six basic emotions are specified with the intensity as very high, high, neutral, low and very low. The visual instrument is shown in FIG.6 as an illustration of a plurality of emotional state presented as a $6 \times 5$ grid wherein each triplet enclosed within parenthesis in a cell of the grid is the RGB value of a color as is commonly understood in visual design. The subject is instructed to choose at least one-color palette that he picks to describe the self-reported emotional state. In case the subject picks 2-3 color palette describing his self-reported emotional state, then the subject is prompted to rank the chosen colors. To calculate the score of assessing the subject with the visual instrument, the set of color palette by the subject are converted into a 6-D vector, $[e_1, e_2, e_3, e_4, e_5, e_6$, where $e_1$ = happy, $e_2$ = surprise, $e_3$ = sad, $e_4$ = disgust, es = fear, $e_6$ = anger. When a subject select $k(<< 30)$ number of colors, namely, $V = \{z_1, z_2, \cdots, z_k\}$ from the list of 30 different color palette presented in the FIG. 6, then the chosen list of words V is converted to an emotion vector. Therefore, $E_v = v_2 ev(V)$, where $E_v$ is the vector representation of the words (V) chosen by the subject. The vector $E_v$ is chosen to represent a self-assessed emotional state of the subject.

A visual score of the pre-emotional state is calculated to assess the subject by administering the visual instrument as:

$$VI_1 = (c_1 e_1 + \ldots \ldots \ldots \ldots c_n e_n)_{pre} \qquad (5)$$

where $e_1$ to $e_n$ are a number of self-reported emotions from the plurality of emotions presented in the visual instrument, and $c_1 = 1$, $c_2 = 0.5$, $c_3 = 0.25$, .......and $= n_{1/2}$ are the half-life of each of the plurality of emotions.

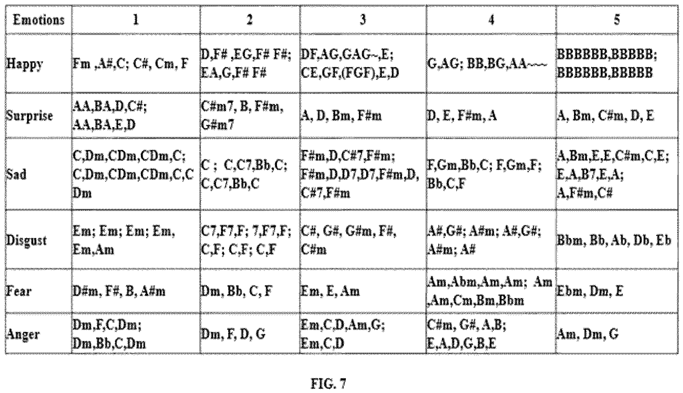

Similarly, the aural instrument is designed to assess present emotional state of the subject. In the method disclosed, the aural instrument is incorporated by acknowledging the fact that the music triggers brain functioning. It increases blood flow to brain regions that generate and control emotions. The limbic system, which is involved in processing emotions and controlling memory, "lights" up when ears perceive music. Therefore the aural instrument as shown in FIG. 7 is administered to the subject to probe and seek the present emotional state of the subject. The aural instrument is designed considering six basic emotions (happy, surprise, sad, disgust, fear and anger). To precisely assess the emotional state of the subject, each of the six basic emotions have assigned a specific set of chords that can define a variation within each emotion. The aural instrument is shown in FIG.7 as an illustration of a plurality of emotional state presented as a $6 \times 5$ grid. The subject is instructed to choose at least one set of chords (i.e a music file) that he picks to describe the self-reported emotional state. In case the subject selects 2-3 music files describing his self-reported emotional state, then the subject is prompted to rank the chosen music files. To calculate the score of assessing the subject with the aural instrument, the music files selected by the subject are converted into a 6-D vector, $[e_1, e_2, e_3, e_4, e_5, e_6$, where $e_1$ = happy, $e_2$ = surprise, $e_3$ = sad, $e_4$ = disgust, $e_5$ = fear, $e_6$ = anger. When a subject select $k(<< 30)$ number of music files, namely, $M = \{z1, z2, \cdots, zk\}$ from the list of 30 different music files presented in the FIG.7, then the chosen list of music files M is converted to an emotion vector. Therefore, $E_M = m_2 ev(M)$, where $E_M$ is the vector representation of the music files (M) chosen by the subject. The vector $E_M$ is chosen to represent a self-assessed emotional state of the subject. The aural score of the pre-emotional state is calculated to assess the subject by administering the aural instrument as:

$$UI_1 = (b_1 e_1 + \ldots \ldots \ldots \ldots b_n e_n)_{pre} \qquad (6)$$

where $e_1$ to $e_n$ are a number of self-reported emotions from the plurality of emotions presented in the visual instrument, and $b_1 = 1$, $b_2 = 0.5$, $b_3 = 0.25$, .......and $b_n = n_{1/2}$ are the half-life of each of the plurality of emotions. Therefore, the first cognitive score is calculated by utilizing the at least one of the linguistic instrument, the aural instrument, and the visual instrument to obtain a linguistic score of the pre-emotional state, a visual score of the pre-emotional state, and an aural score of the pre-emotional state respectively as:

$$\text{First cognitive score} = (LI_1 + UI_1 + VI_1)_{pre} \qquad (7)$$

where $LI_1$ is $(a_1 e_1 + \ldots \ldots \ldots \ldots a_n e_n)_{pre}$, $UI_1$ is $(b_1 e_1 + \ldots \ldots b_n e_n)_{pre}$, and $VI_1$ is $(c_1 e_1 + \ldots \ldots c_n e_n)_{pre}$, and where $e_1$ to $e_n$ are a number of self-reported emotions from a plurality of emotions presented in the linguistic instrument, the aural instrument and the visual instrument, and $a_1 = 1$, $a_2 = 0.5$, $a_3 = 0.25$, .......and $a_n = n_{1/2}$ are the half-life of each of the plurality of emotions.

The first cognitive score is a score of at least one of a linguistic score obtained from the linguistic instrument, an aural score obtained from the aural instrument, or a visual score obtained from the visual instrument. The first cognitive score is a combination of at least two of the cognitive instruments. The first cognitive score is a combination of all three cognitive instruments.

At step 308 of the method 300, the one or more hardware processors 104 are configured to assess a pre-emotional state of the subject based by aggregating the first semiotic score and the first cognitive score. The cognitive score is an aggregation of one or more scores derived from the linguistic instrument, the aural instrument and the visual instrument. The first cognitive score is calculated as per equation (1).

At step 310 of the method 300, the one or more hardware processors 104 are configured to stimulate at least one of a plurality of senses of the subject with a sensory input that triggers the subject to experience a state of emotional variability. The sensory input is generated by exposing the subject to stimulate at least one of a plurality of human senses, and wherein the sensory input is perceived by the subject to experience a state of emotional variability. The sensory input is generated by exposing the subject to stimulate at least one of a plurality of human senses, and wherein the sensory input is perceived by the subject to identify emotional variability. One of the sensory input includes administering one or more odors through nose. Human olfactory system, responsible for sense of smell, begins in the nose and continues into the brain, where odors are processed. Odors are said to influence mood, evoke powerful experiences of pleasure or displeasure, produce alertness or relaxation, and evoke long-forgotten emotional memories. These effects are often said to reflect the dependency of olfaction on parts of the brain involved in emotional experience. Similarly, affective touch activates interoceptive and socio-emotional brain circuits. The affective touch modulates processing of emotion facial expressions. Tactile experiences have potential consequences in emotional processing. Moreover, sensory stimulus through taste is linked to positive and negative emotions. For example, sweet taste has been linked to pleasantness, with the highest valence and arousal ratings, while bitter taste corresponded to anger and disgust; mixed emotions are evoked by salty and sour taste (Robin et al., 2003; Wang et al., 2016). Therefore, at least one sensory stimulus is processed with an intent to alter the present emotional state of the subject. According to an embodiment, the state of emotional variability achieved by stimulating the at least one of a plurality of senses of the subject is utilized as an input to generate an influential marketing strategy for a better customer experience.

At step 312 of the method 300, the one or more hardware processors 104 are configured to receive a second self-reported emotional state of the subject by exposing the subject to the semiotic instrument. The semiotic instrument comprises the plurality of non-verbal cues expressing the plurality of emotions. Each non-verbal cue of the plurality of non-verbal cues is coupled with the strength indicator.

At step 314 of the method 300, the one or more hardware processors 104 are configured to process second self-reported emotional state of the subject using the pre-defined metric to generate a post-emotional semiotic score (Semiotic score$_{(post\text{-}emotional)}$) based on the second self-reported emotional state as:

$$\text{Second semiotic score or } M_{\text{post-emotional}} = \{Si, Wi\} \qquad (8)$$

where $S_{i} = \{MH, H, N, S, MS\}$ and S represents one or more emotional states, such as MH is more happy, H is happy, N is neutral, S is sad and MS is more sad],

W = {0,25,50,75,100} where W represents intensity given to each emotional state.
M = {Si,Wi} [Si - one element of the set Si, Wi - one element of the set W]
M {Si, Wi} - score from the metric
Example: M{H,25} means emotions is quantified as happy with 25% strength.

At step 316 of the method 300, the one or more hardware processors 104 are configured to obtain a second cognitive score of the subject by presenting to the subject at least one cognitive instrument. The response of the subject while interacting with the at least one cognitive instrument is processed to generate the second cognitive score of the subject, and wherein the at least one cognitive instrument is (i) the linguistic instrument, (ii) the aural instrument, and (iii) the visual instrument. Similar to the pre-emotional assessment, the post-emotional assessment is performed by utilizing the at least one of the linguistic instrument, the aural instrument, and the visual instrument to obtain a linguistic score of the post-emotional state, a visual score of the post-emotional state, and an aural score of the post-emotional state.

$$\text{Second cognitive score} = (LI_2 + UI_2 + VI_2)_{\text{post}} \qquad (9)$$

where $LI_2$ is $(a_1e_1 + \dots \dots a_ne_n)_{\text{post}}$, $UI_2$ is $(b_1e_1 + \dots \dots b_ne_n)_{\text{post}}$, and $VI_2$ is $(c_1e_1 + \dots \dots c_ne_m)_{\text{post}}$, and where $e_1$ to $e_n$ are a number of self-reported emotions from a plurality of emotions presented in the linguistic instrument, the aural instrument and the visual instrument, and $a_1 = 1$, $a_2 = 0.5$, $a_3 = 0.25$, ....... and $a_n = n_{1/2}$ are the half-life of each of the plurality of emotions.

At step 318 of the method 300, the one or more hardware processors 104 are configured to assess a post-emotional state of the subject by aggregating the second semiotic score and the second cognitive score as represented in the equation (2).

At step 320 of the method 300, the one or more hardware processors 104 are configured to estimate an altered emotional state of the subject by obtaining a vector difference between the pre-emotional state and the post-emotional state. The vector difference between the pre-emotional state and the post-emotional state is an angular difference calculated as a cosine of an angle between the pre-emotional state and post-emotional state, and is represented as:

$$\text{cosine } (A,B) = (\text{sum (scalar product } (A \cdot B))/ (\text{MOD}(A) \times \text{MOD}(B)) \qquad (10)$$

where,

A is a vector representation of the pre-emotional state,
B is a vector representation of the post-emotional state,

(scalar product $(A \cdot B)$) is $(\text{pre}(a_1 e_1)^* \text{post}(a_1 e_1)) + \ldots\ldots\ldots\ldots\ldots\ldots + (\text{pre}(a_n e_n)^* \text{post}(a_n e_n))$,

$e_1$ to $e_n$ are a number of self-reported emotions from a plurality of emotions presented in the at least one cognitive instrument, and
$a_{1\text{ to }} a_n$ are weights assigned to each self-reported emotions from a plurality of emotions presented in the at least one cognitive instrument.

VALIDATION OF PRE-EMOTIONAL STATE ASSESMENT AND POST-EMOTIONAL STATE ASSESSMENT USING ELECTOENCEPHALOGRAM (EEG) - A TRIANGULATION APPROACH

The sensory stimulus causes alteration in the brain activity. The EEG is a known tool to measure electrical signals in the brain to understand the altered brain activity. Therefore, validation of the pre-emotional state assessment and the post-emotional state assessment is performed using an EEG headset. A commercially available, low-cost, 4-channel wearable EEG band is utilized to acquire continuous EEG data, sampled at 256 Hz. Before providing the sensory stimulus to the subject, the EEG headband is placed on the subject's forehead (closer to the eyebrows); the EEG signal is verified for stability using museLSL live display. The EEG is recorded from AF7, AF8, TP9, and TP10 channels for 30 seconds to assess the pre-emotional state of the subject. Further, as the subject is provided the sensory stimulus and the post-state assessment is performed using the semiotic instrument ad the cognitive instrument, the validation is performed using the EEG. To assess the post-emotional state of the subject wherein the sensory stimulus is believed to alter the brain activity, the EEG is recorded from AF7, AF8, TP9, and TP10 channels for 30 seconds.

According to an embodiment, altering the emotional state of the customers in a shopfloor environment by stimulating the customer with one or more sensory stimulus let better customer experience. One or more atmospheric stimuli in the form of an atmospheric effect of music, odor, and/or color is utilized as an influential marketing with an intent of successful product deployment through an enhanced customer experience. Integration of the semiotic instrument and the at least one cognitive instrument with the low cost, portable EEG results into more holistic assessment of the altered emotional state created by one or more sensory stimulus. Processing of the self-reported emotions as well as physiological reading captured through the EEG provided more accurate assessment of the altered emotional state.

USE CASE: STIMULATING BY AN ODOR ADMINISTRATION AND ASSESSMENT OF AN ALTERD EMOTIONAL STATE

An example scenario of assessing the altered emotional state in a group of human volunteers is provided wherein the altered emotional state is created by exposing the group of human volunteers to an odor and the altered emotional state is identified by the disclosed method utilizing the system 100 is presented below. Also, a validation analysis to estimate the accuracy of the disclosed method done through the EEG is presented below.

The impact of the odor is probed on group of human volunteers via two instruments (semiotic instrument and linguistic instrument), and a parallel EEG analysis is conducted to validate the results of the two instruments. The first two instruments are more of self-reporting by the volunteers. However, self-reporting instruments are known to suffer from biases, hence, to balance the effects, the disclosed invention utilizes at least two such instruments. The validation is done through the EEG headband which measures the physiological impact of the intervention. The EEG signal and the self-assessment data are triangulated and interpreted to arrive at the findings.

Ten volunteers are recruited beforehand by convenient sampling for data collections and specifically instructed not to drink, smoke or take any drugs, including caffeine. They are also asked to come with a head-bath (no shampoo) and told not to eat or drink caffeine an hour before their data collection schedule. The volunteers as well as the person collecting the EEG data are instructed not wear any perfume/deodorants/hair oil/gloves on the days of data collection.

PROTOCOL OF EXPERIMENT:

1. Ten healthy volunteers (4 females and 6 male, with a mean age of ≈ 35 years) arrive and occupies a comfortable chair. All volunteers had a normal nasal anatomy and were not affected by viral cold or nasal block during the data collection
2. Asked to sign a consent form; the data collection process is explained.
3. Handed over a printed copy of the semiotic instrument as shown in FIG. 4 and volunteers are asked to mark their current emotional state which is considered as a pre-emotional state while performing assessment based on the disclosed method.
4. The volunteers are then asked to relax.

(a) A cotton swab is used to block their ears from external noise; an eye mask is used to block external visuals.
(b) EEG headset is placed on the subject's forehead (closer to the eyebrows); the EEG signal is verified for stability using museLSL live display.

5. EEG recorded from AF7, AF8, TP9, and TP10 channels

(a) For 30 sec without any stimuli (pre-stimulus).
(b) Bottle containing the odor is opened and kept at a distance of 3 - 5 cm below the nose of the subject for the next 30 sec(post-stimulus). The volunteers inhales and exhales when odor is presented.

6. The bottle is closed; the EEG signal acquisition is stopped. The EEG headband is removed. The mask is removed, and the subject is asked to dispose of the cotton swab.
7. Repeat Step #3 wherein the volunteers are handed a printed copy of the linguistic instrument as shown in FIG. 5, and volunteers are asked to assess their current emotional state which is considered as a post-emotional state while performing assessment based on the disclosed method.
8. Conducted an open-ended interview with the subject after the end of each session.

Lavender odor is presented to the volunteers. The recording is done in a $5 \times 5 \times 8$ m 3 air-conditioned closed room with a door. After each session, the odor in the room was flushed using an exhaust fan for about 15 min to ensure that there was no residual odor because of the previous data collection process.

The pre-emotional state is calculated as an aggregation of the semiotic score (pre-emotional state) and the cognitive score (pre-emotional state). The semiotic score (pre-emotional state) is generated by processing the response of the subjects to the semiotic instrument. The cognitive score (pre-emotional state)is generated by processing the response of the subjects to the linguistic instrument. Similarly, the post-emotional state is calculated as an aggregation of the semiotic score (post-emotional state) and the cognitive score (post-emotional state). The semiotic score (post-emotional state) is generated by processing the response of the subjects to the semiotic instrument. The cognitive score (post-emotional state) is generated by processing the response of the subjects to the linguistic instrument. The cosine value as a vector difference between the pre-emotional state and the post-emotional state is presented in Table-1. The cosine similarity between the pre-emotional state and the post-emotional state is correlated with differences in the EEG signal collected pre-stimulus and post-stimulus via the odor. The EEG data of all the volunteers are digitally filtered, as is typical in EEG processing, using a band-pass filter of 0.1-60 Hz to remove DC offset; this was followed by passing the signal though a notch filter to remove the line noise present at 50 Hz. The percentage difference of the pre-emotional state and the post emotions state is presented in Table-2. It is observed that sensory stimulus via odor administration altered the emotional state of the subjects and hence resulted into a difference in the electrical activity of the brain resulted into an altered EEG pattern.

Table-1

| | Pre-emotional state assessment | | Post-emotional state assessment | | |
| | Semiotic instrument | Linguistic instrument | Semiotic instrument | Linguistic instrument | |
| | Valence, Arousal | Word vector | Valence, Arousal | Word vector | Cosine |
| Volunteer Identification (ID) | | | | | |
| ID#01 (M) | 5,3 | 1.5,0,0,0, 0,0 | 5,4 | 0,0,0,0,0.5,1 | 0 |
| ID#02 (F) | 4,3 | 0,0,1,0,0,0 | 4,3 | 0,0,1,0,0,0 | 0 |
| ID#03 (F) | 4,3 | 1.5,0,0,0.375, 0,0 | 5,4 | 1.75,0,0,0,0,0 | 0.97 |
| ID#04 (F) | 4,4 | 1.5,0,0,0,0,0 | 5,4 | 1.875,0,0,0,0,0 | 1 |
| ID#05 (M) | 3,3 | 0.03125,0.03125, 0.03125, 0.03125, 0.03125, 0.03125 | 3,3 | 0.03125, 0.03125, 0.03125,0.03125, 0.03125, 0.03125 | 1 |
| ID#06 (F) | 5,3 | 0, 0.1875, 0,1.5, 0, 0.25 | 5,3 | 0, 0.375, 1, 0.5, 0, 0.0625 | 0.46 |
| ID#07 (M) | 5,4 | 1.25, 0, 0.5, 0, 0, 0 | 5,5 | 1.875,0,0,0,0,0 | 0.93 |
| ID#08 (M) | 4,5 | 1.5, 0, 0, 0, 0, 0 | 5,5 | 1.75,0,0,0,0,0 | 1 |
| ID#09 (M) | 4,4 | 1, 0.25, 0, 0.5, 0, 0 | 5,4 | 1.5,0,0,0.25,0,0 | 0.93 |
| ID#010 (M) | 4,3 | 0,1,0, 0,0.75,0 | 4,4 | 1.5,0,0,0,0,0 | 0 |

Table-2

| | EEG Band | Pre-emotional state | Post-emotional state | Percentage difference |
|---|---|---|---|---|
| ID#01 (M) | $\alpha$-AF7 | 1.04 | 0.97 | -6.73 |
| | $\alpha$-AF8 | 1.93 | 1.91 | -1.04 |
| | $\beta$-AF7 | 1.34 | 1.68 | 25.37 |
| | $\beta$-AF8 | 1.77 | 2.23 | 25.99 |
| ID#02 (F) | $\alpha$-AF7 | 3.79 | 2.94 | -22.43 |
| | $\alpha$-AF8 | 1.67 | 1.52 | -8.98 |
| | $\beta$-AF7 | 6.2 | 10.39 | 67.65 |
| | $\beta$-AF8 | 1.46 | 1.68 | 15.07 |
| ID#03 (F) | $\alpha$-AF7 | 1.12 | 0.88 | -21.43 |
| | $\alpha$-AF8 | 1.09 | 1.04 | -4.59 |
| | $\beta$-AF7 | 1.5 | 1.57 | 4.67 |
| | $\beta$-AF8 | 6.72 | 6.99 | 4.02 |
| ID#04 (F) | $\alpha$-AF7 | 2.04 | 1.84 | -9.80 |
| | $\alpha$-AF8 | 2.93 | 2.9 | -1.02 |
| | $\beta$-AF7 | 2.97 | 3.1 | 4.38 |
| | $\beta$-AF8 | 8.41 | 9.36 | 11.30 |

(continued)

| | EEG Band | Pre-emotional state | Post-emotional state | Percentage difference |
|---|---|---|---|---|
| ID#05 (M) | $\alpha$-AF7 | X | X | X |
| | $\alpha$-AF8 | X | X | X |
| | $\beta$-AF7 | X | X | X |
| | $\beta$-AF8 | X | X | X |
| ID#06 (F) | $\alpha$-AF7 | 1.07 | 1.07 | 0.00 |
| | $\alpha$-AF8 | 0.78 | 0.72 | -7.69 |
| | $\beta$-AF7 | 1.83 | 2.65 | 44.81 |
| | $\beta$-AF8 | 1.94 | 2.48 | 27.84 |
| ID#07 (M) | $\alpha$-AF7 | 1.25 | 1.15 | -8.00 |
| | $\alpha$-AF8 | 1.74 | 1.65 | -5.17 |
| | $\beta$-AF7 | 1.63 | 1.74 | 6.75 |
| | $\beta$-AF8 | 2.04 | 2.25 | 10.29 |
| ID#08 (M) | $\alpha$-AF7 | 1.61 | 1.49 | -7.45 |
| | $\alpha$-AF8 | 7.54 | 6.82 | -9.55 |
| | $\beta$-AF7 | 13.36 | 13.79 | 3.22 |
| | $\beta$-AF8 | 19.72 | 21.33 | 8.16 |
| ID#09 (M) | $\alpha$-AF7 | 2.29 | 1.97 | -13.97 |
| | $\alpha$-AF8 | 20.86 | 6.28 | -69.89 |
| | $\beta$-AF7 | 2.48 | 2.68 | 8.06 |
| | $\beta$-AF8 | 4.82 | 5.3 | 9.96 |
| ID#10 (M) | $\alpha$-AF7 | 2.57 | 2.35 | -8.56 |
| | $\alpha$-AF8 | 2.16 | 2.01 | -6.94 |
| | $\beta$-AF7 | 1.65 | 2.07 | 25.45 |
| | $\beta$-AF7 | 2.24 | 2.94 | 31.25 |

The written description describes the subject matter herein to enable any person skilled in the art to make and use the embodiments. The scope of the subject matter embodiments is defined herein and may include other modifications that occur to those skilled in the art. Such other modifications are intended to be within the scope of the present disclosure if they have similar elements that do not differ from the literal language of the present disclosure or if they include equivalent elements with insubstantial differences from the literal language of the embodiments described herein.

Therefore, a method of assessing an altered emotional state under the influence of sensory stimulus is disclosed. The method utilizes a semiotic instrument and one or more cognitive instruments comprising (a) a linguistic instrument, (b) a visual instrument, and (c) an aural instrument to assess the emotional state of the subject. The first assessment of the emotional state is performed before exposing the subject to the sensory stimulus, and the second assessment of the emotional state is performed after exposing the subject to the sensory stimulus. The vector difference between the pre-emotional state and the post-emotional state is calculated to assess the altered emotional state. The disclosed method of interpreting the altered emotional state through the semiotic instrument and the cognitive instrument as well as validating by a physiological assessment through an EEG is a triangulated approach of assessing the altered emotional state. The usefulness of the instrument's utility is established by the co-relation between EEG and the instruments as proven by the experimentation. Hence the two instruments can be solely used as a low-cost measure for detecting emotion change.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1.  A processor implemented method (300) of assessing emotional alteration, the method comprises steps:

    receiving (302), via one or more hardware processors, a first self-reported emotional state of a subject by exposing the subject to a semiotic instrument, wherein the semiotic instrument comprises a plurality non-verbal cues expressing a plurality of emotions, and wherein each non-verbal cue of the plurality of non-verbal cues is coupled with a strength indicator;
    processing (304), via the one or more hardware processors, the first self-reported emotional state of the subject using a pre-defined metric to generate a first semiotic score of the subject;
    obtaining (306), via the one or more hardware processors, a first cognitive score of the subject by processing interactive response of the subject to at least one cognitive instrument.
    assessing (308), via the one or more hardware processors, a pre-emotional state of the subject based by aggregating the first semiotic score and the first cognitive score;
    stimulating (310), via the one or more hardware processors, at least one of a plurality of senses of the subject with a sensory input that triggers the subject to experience a state of emotional variability;
    receiving (312), via the one or more hardware processors, a second self-reported emotional state of the subject by exposing the subject to the semiotic instrument;
    processing (314), via the one or more hardware processors, the second self-reported emotional state of the subject using the pre-defined metric to generate a second semiotic score of the subject;
    obtaining (316), via the one or more hardware processors, a second cognitive score of the subject by processing interactive response of the subject to at least one cognitive instrument;
    assessing (318), via the one or more hardware processors, a post-emotional state of the subject by aggregating the second semiotic score and the second cognitive score; and

estimating (320), via the one or more hardware processors, an altered emotional state of the subject by obtaining a vector difference between the pre-emotional state and the post-emotional state.

2. The method as claimed in claim 1, wherein the at least one cognitive instrument is (i) a linguistic instrument, (ii) an aural instrument, and (iii) a visual instrument.

3. The method as claimed in claim 1, wherein the vector difference between the pre-emotional state and the post-emotional state is an angular difference calculated as a cosine of an angle between the pre-emotional state and post-emotional state, and is represented as:

$$\text{cosine } (A,B) = (\text{sum } (\text{scalar product } (A \cdot B))/ (\text{MOD}(A) \times \text{MOD}(B))$$

where,

A is a vector representation of the pre-emotional state,
B is a vector representation of the post-emotional state,

$$(\text{scalar product } (A \cdot B)) \text{ is } (\text{pre}(a_1 e_1)^* \text{post}(a_1 e_1)) + \ldots + (\text{pre}(a_n e_n)^* \text{post}(a_n e_n)),$$

$e_1$ to $e_n$ are a number of self-reported emotions from a plurality of emotions presented in the at least one cognitive instrument, and
$a_1$ to $a_n$ are weights assigned to each self-reported emotions from a plurality of emotions presented in the at least one cognitive instrument.

4. The method as claimed in claim 1,

wherein the linguistic instrument is designed by steps comprising:

(a) prompting a LLM with a set of words suitable to describe a plurality of emotions;
(b) retrieving a list of words generated by the LLM in response to the set of words prompted;
(c) manually curating the list of words retrieved by the LLM, wherein a subjective assessment of the list of words is performed based on wider understanding and acceptability; and
(d) randomizing the list of words to arrange in a tabular form, and wherein the randomization allows the subject to explore a word-scape and choose one or more words from the table that represents a current emotional state of the subject; and

wherein the first cognitive score is calculated using the linguistic instrument by a three-stage process comprising:

(a) a first stage, wherein the subject is administered with the linguistic instrument;
(b) a second stage, wherein a response is captured from the subject wherein the response comprises (i) one or more words selected from the linguistic instrument that describes the current emotional state of the subject, and (ii) an assigned rank to each word from the one or more words selected from the linguistic instrument to obtain one or more ranked words; and
(c) a third-stage, wherein the one or more ranked words are processed to calculate the cognitive score of the subject.

5. The method as claimed in claim 1,

wherein an electroencephalogram (EEG) associated with the pre-emotional state and the post-emotional state of the subject is processed to identify the altered emotional state of the subject; and
wherein the state of emotional variability achieved by stimulating the at least one of a plurality of senses of the subject is utilized as an input to generate an influential marketing strategy for a better customer experience.

6. A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

receive a first self-reported emotional state of a subject by exposing the subject to a semiotic instrument, wherein the semiotic instrument comprises a plurality non-verbal cues expressing a plurality of emotions, and wherein each non-verbal cue of the plurality of non-verbal cues is coupled with a strength indicator;
process the first self-reported emotional state of the subject using a pre-defined metric to generate a first semiotic score of the subject;
obtain a first cognitive score of the subject by processing interactive response of the subject to at least one cognitive instrument.
assess a pre-emotional state of the subject based by aggregating the first semiotic score and the first cognitive score;
stimulate at least one of a plurality of senses of the subject with a sensory input that triggers the subject to experience a state of emotional variability;
receive a second self-reported emotional state of the subject by exposing the subject to the semiotic instrument;
process the second self-reported emotional state of the subject using the pre-defined metric to generate a second semiotic score of the subject;
obtain a second cognitive score of the subject by processing interactive response of the subject to at least one cognitive instrument;
assess a post-emotional state of the subject by aggregating the second semiotic score and the second cognitive score; and
estimate an altered emotional state of the subject by obtaining a vector difference between the pre-emotional state and the post-emotional state.

7. The system as claimed in claim 6, wherein the at least one cognitive instrument is (i) a linguistic instrument, (ii) an aural instrument, and (iii) a visual instrument.

8. The system as claimed in claim 6, wherein the vector difference between the pre-emotional state and the post-emotional state is an angular difference calculated as a cosine of an angle between the pre-emotional state and post-emotional state, and is represented as:

$$\text{cosine } (A,B) = (\text{sum (scalar product } (A \cdot B)) / (MOD(A) \times MOD(B))$$

where,

A is a vector representation of the pre-emotional state,
B is a vector representation of the post-emotional state,

$$(\text{scalar product } (A \cdot B)) \text{ is } (\text{pre}(a_1 e_1) * \text{post}(a_1 e_1)) + \ldots + (\text{pre}(a_n e_n) * \text{post}(a_n e_n)),$$

$e_1$ to $e_n$ are a number of self-reported emotions from a plurality of emotions presented in the at least one cognitive instrument, and
$a_{1 \text{ to }} a_n$ are weights assigned to each self-reported emotions from a plurality of emotions presented in the at least one cognitive instrument.

9. The system as claimed in claim 6,

wherein the linguistic instrument is designed by steps comprising:

(a) prompting a LLM with a set of words suitable to describe a plurality of emotions;
(b) retrieving a list of words generated by the LLM in response to the set of words prompted;
(c) manually curating the list of words retrieved by the LLM, wherein a subjective assessment of the list of words is performed based on wider understanding and acceptability; and
(d) randomizing the list of words to arrange in a tabular form, and wherein the randomization allows the subject to explore a word-scape and choose one or more words from the table that represents a current emotional state of the subject; and

wherein the first cognitive score is calculated using the linguistic instrument by a three-stage process comprising:

(a) a first stage, wherein the subject is administered with the linguistic instrument;
(b) a second stage, wherein a response is captured from the subject wherein the response comprises (i) one or more words selected from the linguistic instrument that describes the current emotional state of the subject, and (ii) an assigned rank to each word from the one or more words selected from the linguistic instrument to obtain one or more ranked words; and
(c) a third-stage, wherein the one or more ranked words are processed to calculate the cognitive score of the subject.

10. The system as claimed in claim 6,

wherein an electroencephalogram (EEG) associated with the pre-emotional state and the post-emotional state of the subject is processed to identify the altered emotional state of the subject; and
wherein the state of emotional variability achieved by stimulating the at least one of a plurality of senses of the subject is utilized as an input to generate an influential marketing strategy for a better customer experience.

11. One or more non-transitory machine-readable information storage mediums comprising one or more instructions which when executed by one or more hardware processors cause:

receiving a first self-reported emotional state of a subject by exposing the subject to a semiotic instrument, wherein the semiotic instrument comprises a plurality non-verbal cues expressing a plurality of emotions, and wherein each non-verbal cue of the plurality of non-verbal cues is coupled with a strength indicator;
processing the first self-reported emotional state of the subject using a pre-defined metric to generate a first semiotic score of the subject;
obtaining a first cognitive score of the subject by processing interactive response of the subject to at least one cognitive instrument.
assessing a pre-emotional state of the subject based by aggregating the first semiotic score and the first cognitive score;
stimulating at least one of a plurality of senses of the subject with a sensory input that triggers the subject to experience a state of emotional variability;
receiving a second self-reported emotional state of the subject by exposing the subject to the semiotic instrument;
processing the second self-reported emotional state of the subject using the pre-defined metric to generate a second semiotic score of the subject;
obtaining a second cognitive score of the subject by processing interactive response of the subject to at least one cognitive instrument;
assessing a post-emotional state of the subject by aggregating the second semiotic score and the second cognitive score; and
estimating an altered emotional state of the subject by obtaining a vector difference between the pre-emotional state and the post-emotional state.

12. The one or more non-transitory machine-readable information storage mediums as claimed in claim 11, wherein the at least one cognitive instrument is (i) a linguistic instrument, (ii) an aural instrument, and (iii) a visual instrument.

13. The one or more non-transitory machine-readable information storage mediums as claimed in claim 11, wherein the vector difference between the pre-emotional state and the post-emotional state is an angular difference calculated as a cosine of an angle between the pre-emotional state and post-emotional state, and is represented as:

$$\text{cosine}(A,B) = (\text{sum}(\text{scalar product}(A \cdot B)))/(\text{MOD}(A) \times \text{MOD}(B))$$

where,

A is a vector representation of the pre-emotional state,
B is a vector representation of the post-emotional state,

$$\text{(scalar product } (A{\cdot}B)) \text{ is } (pre(a_1e_1){*}post(a_1e_1))+\ldots+(pre(a_ne_n){*}post(a_ne_n)),$$

$e_1$ to $e_n$ are a number of self-reported emotions from a plurality of emotions presented in the at least one cognitive instrument, and
$a_1$ to $a_n$ are weights assigned to each self-reported emotions from a plurality of emotions presented in the at least one cognitive instrument.

14. The one or more non-transitory machine-readable information storage mediums as claimed in claim 11,

wherein the linguistic instrument is designed by steps comprising:

(a) prompting a LLM with a set of words suitable to describe a plurality of emotions;
(b) retrieving a list of words generated by the LLM in response to the set of words prompted;
(c) manually curating the list of words retrieved by the LLM, wherein a subjective assessment of the list of words is performed based on wider understanding and acceptability; and
(d) randomizing the list of words to arrange in a tabular form, and wherein the randomization allows the subject to explore a word-scape and choose one or more words from the table that represents a current emotional state of the subject; and

wherein the first cognitive score is calculated using the linguistic instrument by a three-stage process comprising:

(d) a first stage, wherein the subject is administered with the linguistic instrument;
(e) a second stage, wherein a response is captured from the subject wherein the response comprises (i) one or more words selected from the linguistic instrument that describes the current emotional state of the subject, and (ii) an assigned rank to each word from the one or more words selected from the linguistic instrument to obtain one or more ranked words; and
(f) a third-stage, wherein the one or more ranked words are processed to calculate the cognitive score of the subject.

15. The one or more non-transitory machine-readable information storage mediums as claimed in claim 11,

wherein an electroencephalogram (EEG) associated with the pre-emotional state and the post-emotional state of the subject is processed to identify the altered emotional state of the subject; and
wherein the state of emotional variability achieved by stimulating the at least one of a plurality of senses of the subject is utilized as an input to generate an influential marketing strategy for a better customer experience.

System **100**

Processors(s) **104**

I/O Interface(s) **106**

Database **112**

Memory **102**

EMOTIONAL STATE ASSESSMENT MODULE **110**

Pre-emotional state sub-module **110A**

Post-emotional state sub-module **110B**

Sensory stimuli analysis sub-module **110C**

EEG data processing sub-module **110D**

FIG. 1

100

204

206

Linguistic
instrument

Cognitive instrument

Aural instrument

Visual
instrument

Pre-emotional
state
assessment

Sensory
stimulus
processing

210

212

Post-emotional
state
assessment

202

Semiotic instrument

EEG Data
processing-1

208

EEG Data
processing-2

214

Emotional state identification

FIG. 2

300

receiving a first self-reported emotional state of a subject by exposing the subject to a semiotic instrument, wherein the semiotic instrument comprises a plurality non-verbal cues expressing a plurality of emotions, and wherein each non-verbal cue of the plurality of non-verbal cues is coupled with a strength indicator — 302

processing the first self-reported emotional state of the subject using a pre-defined metric to generate a first semiotic score of the subject — 304

obtaining a first cognitive score of the subject by presenting to the subject at least one cognitive instrument — 306

assessing a pre-emotional state of the subject based by aggregating the first semiotic score and the first cognitive score — 308

stimulating at least one of a plurality of senses of the subject with a sensory input that triggers the subject to experience a state of emotional variability — 310

A

FIG. 3A

300

(A)

receiving a second self-reported emotional state of the subject by exposing the subject to the semiotic instrument — 312

processing the second self-reported emotional state of the subject using the pre-defined metric to generate a second semiotic score of the subject — 314

obtaining a second cognitive score of the subject by presenting to the subject at least one cognitive instrument — 316

assessing a post-emotional state of the subject by aggregating the second semiotic score and the second cognitive score — 318

estimating an altered emotional state of the subject by obtaining a vector difference between the pre-emotional state and the post-emotional state — 320

FIG. 3B

FIG. 4

| Rage | Joyful | Sicken | Amazed | Repulsion |
|------|--------|--------|--------|-----------|
| Disrelish | Mournful | Fright | Pleased | Fury |
| Anxiety | Blissful | Irritation | Grief | Gloomy |
| Cheerful | Phobia | Wonder | Concern | Panic |
| Mad | Sorrowful | Astonishment | Frustration | Shock |
| Stun | Nausea | Delighted | Abhorrence | Depressed |

FIG. 5

| Emotions | Very High | High | Neutral | Low | Very Low |
|---|---|---|---|---|---|
| Happy | (255,103,0) | (255,154,0) | (255,167,0) | (255,150,0) | (255,219,0) |
| Surprise | (37,92,110) | (90,112,127) | (211,169,171) | (228,206,208) | (227,227,227) |
| Sad | (19,39,62) | (40,63,84) | (57,84,109) | (86,123,137) | (99,141,150) |
| Disgust | (115,148,49) | (199,183,38) | (222,192,84) | (54,217,54) | (155,207,93) |
| Fear | (163,140,179) | (140,158,179) | (154,179,140) | (145,173,161) | (173,145,165) |
| Anger | (198,0,0) | (226,0,0) | (255,0,0) | (255,39,39) | (255,103,103) |

FIG. 6

| Emotions | 1 | 2 | 3 | 4 | 5 |
|---|---|---|---|---|---|
| Happy | Fm ,A#,C; C#, Cm, F | D,F# ,EG,F# F#; EA,G,F# F# | DF,AG,GAG~,E; CE,GF,(FGF),E,D | G,AG; BB,BG,AA~~ | BBBBBB,BBBBB; BBBBBB,BBBBB |
| Surprise | AA,BA,D,C#; AA,BA,E,D | C#m7, B, F#m, G#m7 | A, D, Bm, F#m | D, E, F#m, A | A, Bm, C#m, D, E |
| Sad | C,Dm,CDm,CDm,C; C,Dm,CDm,CDm,C,C Dm | C ; C,C7,Bb,C; C,C7,Bb,C | F#m,D,C#7,F#m; F#m,D,D7,D7,F#m,D, C#7,F#m | F,Gm,Bb,C; F,Gm,F; Bb,C,F | A,Bm,E,E,C#m,C,E; E,A,B7,E,A; A,F#m,C# |
| Disgust | Em; Em; Em; Em, Em,Am | C7,F7,F; 7,F7,F; C,F; C,F; C,F | C#, G#, G#m, F#, C#m | A#,G#; A#m; A#,G#; A#m; A# | Bbm, Bb, Ab, Db, Eb |
| Fear | D#m, F#, B, A#m | Dm, Bb, C, F | Em, E, Am | Am,Abm,Am,Am; Am ,Am,Cm,Bm,Bbm | Ebm, Dm, E |
| Anger | Dm,F,C,Dm; Dm,Bb,C,Dm | Dm, F, D, G | Em,C,D,Am,G; Em,C,D | C#m, G#, A,B; E,A,D,G,B,E | Am, Dm, G |

FIG. 7

Europäisches Patentamt
European Patent Office
Office européen des brevets

# EUROPEAN SEARCH REPORT

**Application Number**

EP 25 19 7013

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YANG WANLU ET AL: "Affective auditory stimulus database: An expanded version of the International Affective Digitized Sounds (IADS-E)", BEHAVIOR RESEARCH METHODS, SPRINGER US, NEW YORK, vol. 50, no. 4, 8 March 2018 (2018-03-08), pages 1415-1429, XP036722004, DOI: 10.3758/S13428-018-1027-6 [retrieved on 2018-03-08] * pages 4, 5, 8, * * figure 2 * | 1-15 | INV. A61B5/16 G06Q30/00 G16H10/20 A61B5/00 |
| A | TRILLA IRENE ET AL: "Affective states influence emotion perception: evidence for emotional egocentricity", PSYCHOLOGICAL RESEARCH, SPRINGER, DE, vol. 85, no. 3, 23 March 2020 (2020-03-23) , pages 1005-1015, XP037424608, ISSN: 0340-0727, DOI: 10.1007/S00426-020-01314-3 [retrieved on 2020-03-23] * page 1008 * * figure 2 * | 1-15 | |
| A | ERK STEFANIE M. ET AL: "Effects of mediated social touch on affective experiences and trust", PEERJ, [Online] vol. 3, 6 October 2015 (2015-10-06), page e1297, XP093332646, ISSN: 2167-8359, DOI: 10.7717/peerj.1297 Retrieved from the Internet: URL:https://peerj.com/articles/1297.html> [retrieved on 2025-11-04] * figure 3 * | 1-15 | |

**TECHNICAL FIELDS SEARCHED (IPC)**

A61B
G06Q

-/--

The present search report has been drawn up for all claims

| Place of search | Date of completion of the search | Examiner |
|---|---|---|
| The Hague | 6 November 2025 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt
European
Patent Office
Office européen
des brevets

**EUROPEAN SEARCH REPORT**

**Application Number**

EP 25 19 7013

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JAEGER SARA R ET AL: "Measuring consumers' product associations with emoji and emotion word questionnaires: case studies with tasted foods and written stimuli", FOOD RESEARCH INTERNATIONAL, ELSEVIER, AMSTERDAM , NL, vol. 111, 17 April 2018 (2018-04-17), pages 732-747, XP085428217, ISSN: 0963-9969, DOI: 10.1016/J.FOODRES.2018.04.010 * pages 3, 4 * * figure 2 * * table 4 * | 1-15 | |
| A | ZOU ZHAO ET AL: "A pilot study of measuring emotional response and perception of LLM-generated questionnaire and human-generated questionnaires", SCIENTIFIC REPORTS, [Online] vol. 14, no. 1, 2 February 2024 (2024-02-02), XP093332648, US ISSN: 2045-2322, DOI: 10.1038/s41598-024-53255-1 Retrieved from the Internet: URL:https://www.nature.com/articles/s41598-024-53255-1> [retrieved on 2025-11-05] * abstract * | 1-15 | **TECHNICAL FIELDS SEARCHED (IPC)** |

-/--

The present search report has been drawn up for all claims

| Place of search | Date of completion of the search | Examiner |
|---|---|---|
| The Hague | 6 November 2025 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 25 19 7013

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PRABOWO DWI WAHYU ET AL: "A systematic literature review of emotion recognition using EEG signals", COGNITIVE SYSTEMS RESEARCH, vol. 82, 27 July 2023 (2023-07-27), page 101152, XP093332649, AMSTERDAM, NL ISSN: 1389-0417, DOI: 10.1016/j.cogsys.2023.101152 * abstract * ----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 6 November 2025 | Costa Angeli, M |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421065329